# EUROPÄISCHE PATENTSCHRIFT

(11) **EP 1 032 302 B1**
(45) Veröffentlichungstag und Bekanntmachung des Hinweises auf die Patenterteilung: **14.12.2005**
(21) Anmeldenummer: 98964367.1
(22) Anmeldetag: 20.11.1998
(51) Int. Cl.: A61B 5/0215

(54) **KORREKTUR VON BLUTDRUCKMESSUNGEN BEI INVASIVEN FLÜSSIGKEITSGEFÜLLTEN SYSTEMEN**
CORRECTION OF BLOOD PRESSURE MEASUREMENTS IN INVASIVE LIQUID-FILLED SYSTEMS
CORRECTION DE MESURES DE PRESSION DANS LE CAS DE SYSTEMES INVASIFS REMPLIS D'UN LIQUIDE

(30) Priorität: 21.11.1997 DE 19753183; 04.05.1998 DE 19820844
(43) Veröffentlichungstag der Anmeldung: 06.09.2000
(73) Patentinhaber: Deutsches Herzzentrum Berlin, 13353 Berlin (DE)
(72) Erfinder: WELLNHOFER, Ernst, D-10589 Berlin (DE)
(74) Vertreter: Ninnemann, Detlef
(86) Internationale Anmeldenummer: PCT/DE1998/003486
(87) Internationale Veröffentlichungsnummer: WO 1999/026531

(56) Entgegenhaltungen:
- WO-A-90/11043
- WO-A-96/04842
- DE-A- 3 927 990
- US-A- 4 232 373

## Beschreibung

Die Erfindung betrifft eine Vorrichtung zur Korrektur von Messwertverfälschungen bei invasiven Druckmessungen mit einem flüssigkeitsgefüllten System, bei denen der gemessene Druck über das flüssigkeitsgefüllte System zu einem externen Druckwandler geleitet wird, der das Drucksignal in ein elektrisches Signal umwandelt.

Im Zusammenhang mit der invasiven Druckmessung werden seit Jahrzehnten flüssigkeitsgefüllte Systeme zur intravenösen und intraarteriellen Druckmessung eingesetzt. Häufige Anwendung finden solche, auch als Katheter bezeichneten Systeme in der invasiven Kardiologie, der Intensivmedizin und in der Anästhesie, wo sie zur exakten Druckmessung eingesetzt werden. Ein Einsatz ist insbesondere sinnvoll für Impedanzmessungen am arteriellen Gefäßsystem oder Ableitungen des Drucks nach der Zeit (dp/dt) zur Messung der isovolumischen Kontraktionskraft oder von Relaxationsstörungen der Herzkammern. Hierfür müssen Resonanzen der Originaldrucksignale bis etwa 30 Hz originalgetreu, also phasen- und amplitudengetreu, analysiert werden können.

Bei der invasiven Katheterdiagnostik erfolgt die Druckmessung an einem bestimmten Ort im Kreislauf über ein flüssigkeitsgefülltes System mit einem extern (d.h. außerhalb des Patientenkörpers) angebrachten Druckwandler. Abhängig von der Länge, dem Querschnitt, dem Aufbau und den elastischen Materialeigenschaften dieser Systeme kommt es zu unterschiedlichen Resonanzen, Dämpfungen und Energieverlusten des Druckeingangssignals an der Katheterspitze.

Aus der US-4 232 373 ist ein Korrekturverfahren für Messdaten eines flüssigkeitsgefüllten Herzkatheters bekannt, bei dem das periodisch aufgenommene Signal in ein elektrisches Signal umgewandelt, digitalisiert und verzweigt wird. Ein Teil des Signals wird zunächst einer Korrektureinheit und anschließend einem Filter zugeleitet, während der andere Teil unkorrigiert und verzögert dem Filter zugeleitet wird. In dem Filter werden die beiden Signalteile zusammengeführt, und das korrigierte Signal wird ausgegeben.

In dem Manuskript "Characterization of laser-induced pressure transients by means of piezoelectric PVDF-films" von S. Lohmann et al.; Proc.SPIE 2624; 83-92; (1995) wird unter anderem die Korrektur von laserinduzierten Druckwellen in piezoelektrischen Folien beschrieben. Dabei wird die Korrektur eines von der Folie abgegebenen Spannungssignals mittels einer Fouriertransformation beschrieben, bei der das Signal in den Frequenzbereich transformiert wird und im Frequenzbereich mittels eines in einem Algorithmus errechneten Korrekturwertes korrigiert wird. Anschließend wird eine Rücktransformation in den Zeitbereich durchgeführt.

Um Verfälschungen entlang des Übertragungsweges zu vermeiden, wurde der Druckwandler in die Spitze des Katheters integriert und das gewandelte Signal über eine elektrische Leitung aus dem Körper herausgeführt. Diese Lösung ist als Tipdrucksensorkatheter bekannt. Nachteilig an dieser Form der Druckmessung ist, dass Tipdrucksensorkatheter sehr teuer sind und bezüglich der Form und Größe nur eine sehr begrenzte Palette von Variationen aufweisen. Diese Lösung konnte sich deshalb nur auf dem wissenschaftlichen Sektor begrenzt etablieren.

Eine weitere Möglichkeit zur Kompensation von Messwertverfälschungen besteht darin, das System als eine einfache erzwungene Schwingung (forced oscillation) im physikalischen Sinn zu betrachten und eine Korrektur der Übertragungsfunktion dieses Systems 2. Ordnung nach Ermittlung der Resonanzfrequenz und des Dämpfungskoeffizienten mittels einer analogen elektrischen Schaltung oder eines entsprechenden numerischen Algorithmus durchzuführen. Die Nachteile dieses Ansatzes sind, daß die Betrachtung als System 2. Ordnung eine starke Vereinfachung der tatsächlichen Physik des Systems darstellt, in dem insbesondere bei komplexeren Systemen Mehrfachresonanzen auftreten können. Die Übertragungsfunktion ist für jedes konkrete System, auch bei üblichen und häufigen Veränderungen wie Austausch des Katheters im System, im Prinzip neu zu bestimmen, wobei die Bestimmung der Übertragungsfunktion mittels Spültest oder Rechtecktest am Patienten problematisch ist. Die Übertragungsfunktion ist ferner abhängig von der Elastizität des Systems und die wiederum ist abhängig vom Füllungsdruck, den in der Flüssigkeit gelösten Gasen und Materialeigenschaften des Systems. Schließlich ist die Bedienung dieser Systeme sehr kompliziert.

Weitere auf dem Markt eingeführte Vorrichtungen sind als speziell konfigurierte und durch in vitro Testuntersuchungen flüssigkeitsmechanisch optimierte, vollständige Systeme mit einem Druckwandler, Schlauch, Dreiwegehahn, Hahnenbank, Katheter und eventuell Dämpfungsglied ausgebildet. Nachteilig an diesen Vorrichtungen ist, dass der Testaufwand sehr hoch ist und dass in der invasiven Kardiologie eine extrem große Vielfalt von Systemen zum Einsatz kommt, die den Einsatz dieser Vorrichtungen limitiert. Weiterhin ist ein Abschalten dieser Dämpfung zum Ausschluss einer Dämpfung durch Blut oder Luft im System nicht möglich. Die Sehgewohnheiten des Katheterpersonals verbinden mit Dämpfung ein unzureichend gespültes System und würden ein solch gedämpftes System leicht missdeuten.

Aufgabe der vorliegenden Erfindung ist es, eine bezüglich der Korrektur von Messwertverfälschungen verbesserte Vorrichtung zur invasiven Druckmessung mit flüssigkeitsgefüllten Systemen bereitzustellen, die kostengünstig und vielseitig einsetzbar sind.

Erfindungsgemäß wird die Aufgabe durch eine Vorrichtung mit den Merkmalen des Anspruches 1 oder des Anspruchs 7 gelöst.

Die erfindungsgemäße Vorrichtung erlaubt eine phasen- und amplitudengetreue Korrektur des Druckverlaufes durch die Auswertung und Bearbeitung des Signals mittels der digitalen Fourieranalyse, wobei keine Fouriertransformation eines Signals festgelegter Länge eingesetzt wird, sondern mit variablen Signallängen gearbeitet wird. Auf diese Weise kann die optimale Segmentlänge für die anschließende Korrektur bestimmt werden, bei der ein minimaler Fehler auftritt.

Die Vorrichtung ist für eine Vielzahl von Systemen einsetzbar, wodurch der finanzielle und apparative Aufwand bei invasiven Druckmessungen verringert wird. Weiterhin entfallen bauartbedingte Restriktionen, so dass die für den Patienten optimalen Systeme bzw. Katheter eingesetzt werden können, ohne auf entsprechende Genauigkeit verzichten zu müssen.

Die Ausgabe an verschiedene Auswerte- bzw. Anzeigeeinheiten ermöglicht eine schnelle und umfassende Auswertung der Daten. Eine Korrektur der Signale ist sowohl online als auch offline möglich.

Zur Bestimmung der Segmentlänge des zu korrigierenden Signals wird ein Vergleich der Abweichung der Rücktransformierten von dem Originalsignal auf der Grundlage der Variation der Länge eines Basissignals durchgeführt. Ausgehend von einer vorgegebenen Basissignallänge wird ein Vergleich der Rücktransformierten des Basissignals mit dem Originalsignal durchgeführt. Dabei wird eine Abweichung bzw. ein Fehler festgestellt, der sich in Abhängigkeit von der gewählten Signallänge verändert. Die Signallänge wird nun schrittweise vergrößert oder verringert, je nachdem von welcher Basissignallänge ausgegangen wird. Erreicht der Fehler einen vorgegebenen Wert, d.h. wird eine bestimmte Genauigkeit erreicht, wird die Variation der Segmenttänge abgebrochen, um den Rechenaufwand zu reduzieren. Eine optimale Segmentlänge ist dann gefunden, wenn das Minimum der Abweichung der Rücktransformierten von dem Originalsignal ermittelt wurde.

Als günstig hat sich herausgestellt, dass bei der Variation der Basissignallänge von einer Mindestlänge ausgegangen wird, die schrittweise vergrößert wird. Verringert sich die Abweichung bei einer Vergrößerung der Segmentlänge, wird die Transformationsroutine mit der Fehlerermittlung wiederholt, bis der vorgegebene Wert der Abweichung oder das Minimum erreicht ist. Die so gefundene Segmentlänge bzw. Kurvenlänge ist optimal für die Fouriertransformation, da sich das gemessene Signal nahezu komplett in harmonische Schwingungen zerlegen lässt und der Fehler minimal ist. Als Mindestlänge ist ein Wert anzunehmen, der kleiner als die Länge eines Herzschlages ist. Als ein günstiger Wert hat sich eine Mindestsignallänge von 0.3 Sekunden herausgestellt.

Zur schnelleren Ermittlung des optimalen bzw. vorgegebenen Wertes wird die Schrittweite der Segmentlängenänderung proportional zu der Abweichung der Rücktransformierten von dem Originalsignal variiert. Bei einem kleinen Fehler wird eine entsprechend kleine Veränderung vorgenommen, da sich die Segmentlänge bereits in der Nähe des Optimums befindet und durch eine geringe Schrittweite eine möglichst hohe Auflösung angestrebt wird. Bei einem großen Fehlerwert gilt entsprechendes umgekehrt.

Eine Variante der erfindungsgemäßen Vorrichtung erlaubt eine phasen- und amplitudengetreue Korrektur des Druckverlaufes durch die schlagweise Auswertung und Bearbeitung des Signals mittels der digitalen Fourieranalyse. Andere Korrekturvorrichtungen sind nur unzureichend in der Lage, die unterschiedlichen Frequenzen der Herzschläge zu berücksichtigen.

Für die Korrektur der aufgenommenen Signale bei der schlagweisen Analyse ist es sehr wichtig, dass die Länge des Herzschlages bekannt ist, da nur so exakt ein Schlag bearbeitet werden kann. Die Schlaglänge wird erfindungsgemäß über eine Autokorrelationsfunktion und ihre erste Ableitung nach der Zeit berechnet. Optional ist eine Vorfilterung mit einem Tiefpassfilter mit einer hohen Grenzfrequenz von 30 -40 Hz vorgesehen, um eventuelle Störungen des Wechselstromnetzes auszuschließen:

In einer vorteilhaften Ausgestaltung der Erfindung werden die Korrekturdaten, die auf der Grundlage von Referenzdruckmessungen ermittelt wurden, aus einer Korrekturdatensatzmatrix abgerufen, wodurch eine Vielzahl von Datensätzen schnell und einfach zugänglich gemacht wird. Um die Zahl der empirisch ermittelten Korrekturdatensätze in einer wirtschaftlich vertretbaren Größenordnung zu halten, wird bei Fehlen des exakt passenden Datensatzes eine Interpolation zwischen den nächstliegenden Datensätzen durchgeführt.

Um ein möglichst zutreffendes korrigiertes Signal zu erhalten, ist sowohl eine Phasen- als auch eine Amplitudenkorrektur vorgesehen, wobei es sich als vorteilhaft erwiesen hat, eine Phasenkorrektur des Signals nur an den Stellen durchzuführen, wo dass Signal eine Amplitude aufweist.

Zur Ermittlung der Korrekturdatensätze werden in einer Ausgestaltungsform der Erfindung die Katheterspitze in eine druckbeaufschlagbare Vorrichtung eingeführt und diese Vorrichtung wird mit unterschiedlichen Mitteldrücken und Frequenzen beaufschlagt. In getrennten Messungen wird der Mitteldruck in definierten äquidistanten Schrittweiten und die niedrigste Frequenz (Grundfrequenz) des Frequenzgemisches in ebenfalls definierten äquidistanten Schrittweiten variiert. Diese Einstellungen ergeben ein Mitteldruck-Frequenz-Koordinatengitter, was die Basis für die Korrekturdatensatzmatrix darstellt. Alternativ dazu wird die Übertragungscharakteristik mittels eines weißen Frequenzrauschens bestimmt und die Korrektur erfolgt mittels Dekonvolution des Ausgangssignals mit der Übertragungsfunktion. Eine Referenzdruckmessung erfolgt mit einem anderen Meßsystem, vorzugsweise mit einem Tipdrucksensorkatheter.

Es hat sich bezüglich des Rechenaufwandes und der Korrekturergebnisse als günstig herausgestellt, dass zur Ermittlung der Korrekturdatensätze für die Systemanregung ein definiertes Signal in Gestalt eines Frequenzgitter verwendet wird. Basierend auf einer Grundschwingung, die aus rechentechnischen Gründen vorteilhafterweise im Bereich zwischen 0,2 und 3 Hz liegt, wird das System mit äquidistanten harmonischen Oberschwingungen angeregt. Aus einer festgelegten Obergrenze ergibt sich somit die Anzahl der erforderlichen Anregungsfrequenzen. Als eine physiologisch sinnvolle Obergrenze der Anregungsfrequenz haben sich 40 Hz herausgestellt.

In getrennten Messungen wird der Mitteldruck in definierten äquidistanten Schrittweiten variiert. Diese Einstellungen ergeben einen Satz von Korrekturdatensätzen für verschiedene Mitteldrücke. Eine Referenzdruckmessung erfolgt, wie gehabt, mit einem anderen Meßsystem.

Um eine Übereinstimmung der Spektrallinien des zu korrigierenden Signals mit denen des Korrekturdatensatzvektors zu erreichen, wird das Drucksignalsegment so oft vervielfacht, bis sich ein Verhältnis zwischen der Abtastrate und der Kurvenstücklänge ergibt, das der Auflösung des Korrekturdatensatzes entspricht. Bei Nichtentsprechen der Auflösung wird zweckmäßigerweise das nächst kleinere Verhältnis zwischen der Abtastrate und der Kurvenstücklänge eingestellt und die Zuordnung zu den Spektrallinien des Korrekturdatensatzes erfolgt durch Aufrunden zur nächsten entsprechenden Linie.

Da ein Druckwandler in der Regel kein ausreichend starkes Signal abgibt, ist zwischen dem Druckwandler und dem Analog/Digitalwandler ein Verstärker vorgesehen. Der Druckwandler wird über eine Versorgungsleitung der Signalverarbeitungs- und -analyseeinheit angesteuert und mit der erforderlichen Betriebsspannung versorgt.

Für eine zuverlässige Korrektur der Signale ist es notwendig, dass die Signalanalyse- und -verarbeitungseinheit die jeweiligen Korrekturdatensätze richtig zuordnet. Da die verschiedenen Systeme unterschiedlich ausgelegt sind, verschiedene Resonanzfrequenzen aufweisen und durch Anbauteile stark verändert werden können, wird vor der Messwertaufnahme eine Systemidentifikation über eine Testsignalantwort durchgeführt. Ein definiertes Signal wird vorzugsweise von einem Referenzdruckgeber (Kalibrator) an der Spitze des Katheters in Richtung Druckwandler ausgesendet und die Systemantwort wird mit experimentell gefundenen Systemantworten verglichen. Auf diese Art können eine Klassifikation vorgenommen und Informationen darüber gewonnen werden, um welches System es sich handelt bzw. welche Korrekturdatensätze für das betreffende System geeignet sind. Denkbar ist auch, dass ein Signal von dem Druckwandler in Richtung Katheterspitze ausgesendet und die Signalantwort mit experimentell gefundenen Systemantworten verglichen wird.

Es hat sich bei der schlagweisen Analyse als vorteilhaft erwiesen, dass die Grundfrequenz mittels einer Kombination einer Verteilungsanalyse von Maxima von Autokorrelationsfunktionen variierender Länge mit der Analyse der Minima und Maxima der Kurve bestimmt wird. Gerade bei der online-Bestimmung der Grundfrequenz ist es zweckmäßig, die Autokorrelationsfunktion mit wachsender Länge zu wiederholen und alle ersten Maxima der Autokorrelationsfunktionen größer werdender Länge zu sammeln. Anschließend wird mittels einer Verteilungsanalyse das am häufigsten vorkommende Maximum ermittelt.

Mit Vorteil wird zur Ermittlung der Länge des flüssigkeitsgefüllten Systems, d.h. der Signallaufzeit, eine Kreuzkorrelation von Drucksignal und Patienten-EKG durchgeführt. In einer Variante wird die Systemidentifikation automatisch durchgeführt.

Neben einer Einordnung von Katheter und Schlauchsystem ist mit Vorteil eine Identifikation der unterschiedlichen Druckwandler und eine entsprechende Berücksichtigung bei der Auswahl der Korrekturdatensätze vorgesehen. Da die jeweiligen Druckwandler typen- bzw. bauartbedingt die Drucksignale verschieden umsetzen, unterschiedliche Betriebsspannungen benötigen und individuell angesteuert werden müssen, ist eine solche Anpassung vorteilhaft, um die Messwertverfälschung möglichst gering zu halten und eine korrekte Ansteuerung durchzuführen.

Als zusätzliche Kontrolle und um den Erfahrungsschatz des Bedienpersonals zu nutzen, ist eine manuelle Interaktion bei der Systemidentifikation vorgesehen, so dass ergänzend oder abweichend von der errechneten Option eine Auswahl beziehungsweise eine Eingabe vorgenommen werden kann.

Die Systemübertragungseigenschaften des flüssigkeitsgefüllten Systems korrelieren mit der Elastizität des Katheter- und des Leitungssystems. Abhängig von den Materialeigenschaften kann eine unterschiedliche Vorspannung durch den im System herrschenden mittleren Binnendruck die Systemübertragungseigenschaften deshalb wesentlich verändern. Eine laufende Messung des Mitteldrucks ist deshalb Teil der automatischen Signalanalyse. Die Auswahl der Korrekturdatensätze erfolgt abhängig vom Mitteldruck.

Für eine zuverlässige Korrektur des Drucksignals ist es von Vorteil, wenn sog. Artefakte erkannt werden. Dies geschieht auf der Basis der ermittelten Systemidentifikation. Zu große Abweichungen werden erkannt und nicht berücksichtigt. Bei einer Variante der Vorrichtung wird zusätzlich zu der Korrektur des Drucksignals eine Artefaktidentifikation und -elimination mittels kurzer Autokorrelation durchgeführt. In der Autokorrelationsfunktion lassen sich Störungs-Spikes im Druckverlauf automatisch erkennen und lokalisieren. Eine Interpolation der Kurve an der Stelle des Spikes beseitigt die Störung.

Zusätzlich zu den beschriebenen Korrekturinstrumenten kann gegebenenfalls eine Formanalyse des Drucksignals unter Berücksichtigung von höheren harmonischen Grundschwingungen durchgeführt werden, so dass bei der Erstellung und Auswahl der Korrekturdatensätze die Vorrichtung eine entsprechend verfeinerte Analyse durchführen kann.

In einer weiteren Ausgestaltung der Erfindung ist eine wahlweise Ausgabe des untransformierten Signals vorgesehen, wodurch dem Bedienpersonal ermöglicht wird, die mechanische Dämpfung durch Blutgerinnsel oder kleine Luftblasen zu erkennen.

Zweckmäßigerweise sind statische Kalibrieroptionen vorgesehen, die die Bedienung vereinfachen, beziehungsweise leichter vergleichbare Ergebnisse oder Signalverläufe liefern. Durch einen Nullpunktabgleich wird der gemessene Druck als Nullpunkt angenommen und dient als Basis für das Druckmonitoringsystem, das die Signale anzeigt. Auf diese Weise werden untereinander vergleichbare Anzeigen möglich, ohne dass beispielsweise Blutdruckschwankungen und systembedingte Offsets zwischen verschiedenen Messungen berücksichtigt werden müssen. Zur Überprüfung der Verbindung zwischen Signalanalyse- und -verarbeitungseinheit und Druckmonitoringsystem und zur Überprüfung der Kalibrierung kann ein Referenzdruck (z.B. 100 mmHg im Gerätemenü einstellbar) an das Druckmonitoringsystem gesendet werden. Analog zum Referenzdruck können verschiedene, gespeicherte Druckkurven als Testsignal an das Druckmonitoringsystem gesendet werden.

In einer weiteren Ausgestaltungsform der Erfindung wird das Signal nachgefiltert beziehungsweise nachkorrigiert, um Störsignale zu entfemen und einen möglichst unverfälschten Signalverlauf zu haben. Eine solche Nachkorrektur wird vorzugsweise auf der Basis der ersten Ableitung des korrigierten und gegebenenfalls geglätteten Drucksignals nach der Zeit durchgeführt. Für eine Nachfilterung bieten sich Frequenz- bzw. Mittelwertfilter an.

In einer vorteilhaften Ausgestaltung der Erfindung wird eine automatische Anpassung an Änderungen des Resonanzverhaltens des Systems infolge von Druckänderungen durchgeführt. Die Blutdruckänderungen können beispielsweise durch kreislaufbedingte Reaktionen oder Medikamente hervorgerufen werden, wobei die Änderungen des Resonanzverhaltens systemspezifischer Natur sind. Die entsprechenden Größen werden kontinuierlich ermittelt und der Signalanalyse- und -verarbeitungseinheit laufend zugeführt, weiche die Änderungen bei der Auswahl der Korrekturdatensätze berücksichtigt.

Eine Variante weist bevorzugt zwischen der Signalanalyse- und -verarbeitungseinheit und der Ausgabeeinheit eine Schnittstelle auf, die als Digital/Analogwandler, Verstärker und/oder einen Adapter ausgebildet ist, so dass das korrigierte Signal einem Monitorsystem zugeführt, verstärkt und/oder in digitalisierter Form verbleibend einem Rechner bzw. Computer übermittelt werden kann.

Vorteilhafterweise ist in einem Speicher der Signalanalyse- und -verarbeitungseinheit eine Korrekturdatensatzmatrix abgelegt, die aus experimentellen Referenzdruckmessungen ermittelte Korrekturfaktoren enthält. In Verbindung mit den entsprechenden Datenverarbeitungsprogrammen können dann die jeweiligen oder interpolierten Korrekturvektoren ausgewählt, gegebenenfalls interpoliert und mit dem digitalisierten Drucksignal verknüpft werden.

Normalerweise wird mit einer Dämpfung ein unzureichend gespültes System verbunden. Um die bisherigen Erfahrungen zu nutzen, weist die Vorrichtung vorteilhafterweise einen Signalausgang für das unkompensierte Signal auf, damit das Bedienpersonal die Möglichkeit hat, die korrigierten Drucksignale mit den Signalen in Reinform zu vergleichen und so eine Kontrolle über die durch die Vorrichtung ausgeführte Korrektur zu haben.

Damit die Vorrichtung Blutdruckschwankungen bei der Korrektur berücksichtigen kann, ist in einer Weiterbildung der Erfindung eine Vorrichtung zu deren Messung vorgesehen, wobei die ermittelten Messwerte Einfluss auf die Korrekturdatensatzauswahl nehmen.

Anhand von in der Zeichnung dargestellten Ausführungsbeispielen wird nachfolgend die Erfindung näher erläutert. Es zeigen:
- **FIG. 1** -: einen Prinzipaufbau für die Messwertkorrektur,
- **FIG. 2 -**: einen Prinzipaufbau für die Korrekturdatensatzerstellung sowie
- **FIG. 3 -**: eine Darstellung zur Bestimmung der Grundschwingungslänge.

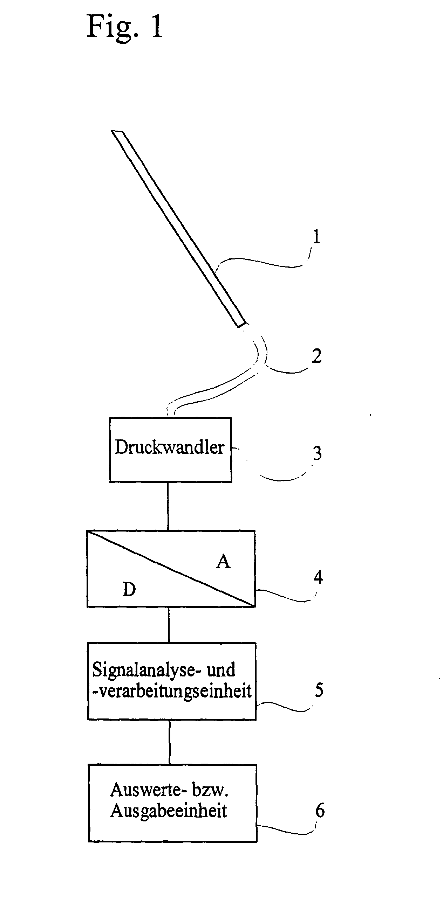

Figur 1 zeigt einen prinzipiellen Aufbau einer invasiven Druckmessung mittels eines flüssigkeitsgefüllten Systems. Dabei wird ein so genannter Katheter 1 durch das venöse oder arterielle System eines Patienten in die Nähe der Stelle bewegt, an der der Druck gemessen werden soll. Um den Patienten möglichst wenig durch den Katheter 1 zu beeinflussen, weist dieser möglichst geringe Abmessungen auf. Der Katheter 1 selbst besteht aus einem elastischen Material und ist schlauchartig ausgebildet. An der Spitze des flüssigkeitsgefüllten Katheters 1 befindet sich eine Öffnung, durch die Druckimpulse aufgenommen und durch den Katheter 1 und eine ebenfalls flüssigkeitsgefüllte Leitung 2 bis zu einem Druckwandler 3 weitergeleitet werden.

Der Druckwandler 3 erzeugt in Abhängigkeit von den Druckimpulsen elektrische Signale, die entsprechend dargestellt bzw. ausgewertet werden können. Eine solche Vorrichtung ist prinzipiell seit längerem bekannt. Eine eventuelle Korrektur der Übertragungsfunktion dieses Systems zweiter Ordnung erfolgte nach Ermittlung der Resonanzfrequenz und des Dämpfungskoeffizienten mittels einer analogen elektrischen Schaltung oder eines entsprechenden numerischen Algorithmus.

Um die bei der Verwendung der oben beschriebenen Methode auftretenden Messverfälschungen, die im Bereich von bis zu 40% liegen, wirksam zu korrigieren, wird bei der erfindungsgemäßen Vorrichtung ein Analog/Digital-Wandler 4 zwischen dem Druckwandler 3 und der Signalanalyse- und -verarbeitungseinheit 5 angeordnet, der die analogen Signale des Druckwandlers 3 in digitale, an den Eingang der Signalanalyse-und -verarbeitungseinheit 5 angelegte Signale umsetzt. Innerhalb der Signalanalyse- und -verarbeitungseinheit 5 werden die gemessenen Daten auf der Basis einer digitalen Fourieranalyse mit Korrekturfaktoren beaufschlagt und an die Ausgabe- bzw. Auswerteeinrichtung 6 weitergeleitet.

Vor der Korrektur der Signale wird eine Gesamtsystemidentifikation des mechanischen Teils des Systems durchgeführt. Zunächst findet eine manuelle oder automatische Identifikation des angeschlossenen Druckwandlers 3 statt. Anschießend wird ein Testsignal in Form eines Druckimpulses ausgesendet, vorzugsweise erzeugt von einem Kalibrator. Als Alternative wird die Impulserzeugung von einem Druckwandler 3 geleistet. Anhand der Signalantwort werden Parameter des Katheter-Leitungs-Systems ermittelt, auf deren Grundlage dann eine Auswahl der Korrekturdatensätze erfolgt. Da bei der Vielzahl der Komponenten, die bei der invasiven Druckmessung eingesetzt werden, und der Vielzahl der Parameter ein exakt passender Korrekturdatensatz nicht immer zur Verfügung steht, werden aus den vorliegenden Datensätzen mittels Interpolation die benötigten Werte ermittelt und zur Korrektur bereitgestellt.

Die digitalisierten und mit korrigierten Fourierkoeffizienten beaufschlagten Signale werden von der Signalanalyse- und -verarbeitungseinheit 5 zu einer Anzeige- bzw. Auswerteeinheit 6 übermittelt, wobei eine Anzeige sowohl auf einem Monitorsystem als auch auf einem Ausdruck erfolgen kann. Je nach Standard des Monitors, werden die Signale zunächst einem Digital/Analog-Wandler zugeführt und anschließend ausgegeben oder direkt einem Monitor überspielt, der digitale Signal verarbeiten kann. Gegebenenfalls müssen die Signale noch dergestalt aufbereitet werden, dass ein für die Darstellung geeignetes Format vorliegt.

Eine andere Möglichkeit besteht in der Übermittlung der Daten an einen Computer, der diese speichert und auswertet. In diesem Fall werden die Daten nicht in einem Digital/Analog-Wandler bearbeitet sondern werden direkt von der Korrektur weitergeleitet.

Es besteht weiterhin die Möglichkeit, die Korrektur nicht online durchzuführen, sondern die Daten abzuspeichern und zu einem späteren Zeitpunkt auszuwerten oder zu korrigieren. Voraussetzung dafür ist das Vorhandensein der systemspezifischen Daten sowie der Informationen über die Messbedingungen, damit nachfolgend eine zutreffende Auswahl der Korrekturdatensätze erfolgen kann. Die Daten werden dafür vorteilhafterweise direkt nach dem Druckwandler 3 aufgenommen und auf einem geeigneten Speichermedium, beispielsweise einer CD oder Diskette, abgelegt.

In einer Variante der Erfindung ist eine Ausgabemöglichkeit für das unkorrigierte Signal vorgesehen, damit die Möglichkeit besteht, die korrigierten Signale mit den unkorrigierten Signalen zu vergleichen. Einerseits werden dadurch die Sehgewohnheiten des Bedienpersonals nicht einer vollständigen Änderung unterworfen, andererseits findet eine Kontrolle der durch die Vorrichtung vorgenommenen Korrektur statt. Das Vorhandensein von Luftblasen im flüssigkeitsgefüllten System kann beispielsweise am unkorrigierten Signal von geschulten Bedienungspersonen erkannt werden, so dass entsprechende Maßnahmen ergriffen werden können. Die Abzweigung des Signals kann sowohl vor als auch nach dem Analog/Digitalwandler 4 erfolgen, wobei ein Vorschalten eines Verstärkers zweckmäßig ist, damit ein ausreichend starkes Signal zur Verfügung steht.

Vor der eigentlichen Messung wird üblicherweise ein Abgleich des zu messenden Druckes gegenüber dem Luftdruck durchgeführt, wobei ein üblicherweise am Druckwandler vorgesehener Dreiwegehahn betätigt wird. An der Signalanalyse- und -verarbeitungseinheit 5 ist ein Betätigungselement vorgesehen, mit dessen Betätigung der zu messende Druck als Nullpunkt angenommen wird und als Basis für die weitere Messung und Ausgabe dient.

Zur Überprüfung der Verbindung zwischen der Signalanalyse- und -verarbeitungseinheit 5 und der Ausgabeeinheit 6 sowie zur Überprüfung der Kalibrierung werden ein Referenzdrucksignal oder verschiedene gespeicherte Druckkurven an die Ausgabeeinheit 6 gesendet. Aus der Differenz zwischen dem Sollwert- und dem Istwert-Signal kann die Abweichung und die vorzunehmende Kompensation bestimmt werden. Soll die gesamte Messkette überprüft werden, kann statt eines Patienten-Drucksignals ein Referenzdrucksignal angeschlossen werden und gegebenenfalls können notwendige Offset- und Linearitätskorrekturen an der Signalanalyse- und -verarbeitungseinheit 5 für jeden Kanal durchgeführt werden.

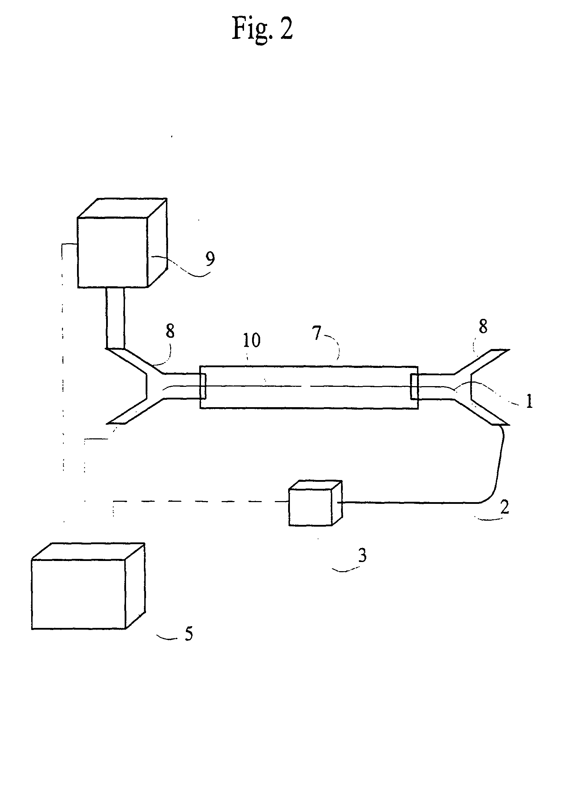

Ein Prinzipaufbau für die empirische Ermittlung der Korrekturdatensätze ist in der Figur 2 dargestellt. Zur Ermittlung der Eigendynamik eines Systems und somit der Korrekturdatensätze, wird ein mit Flüssigkeit gefülltes und entlüftetes Rohr 7 verwendet. Am Rohr 7 befindet sich je ein Anschluss 8 für die Befüllung, die Entlüftung, die Referenzdruckmessung mittels eines Tipdrucksensorkatheters 10 und die Kathetereinführung (Testsystem) sowie eine Vorrichtung für die Druckerzeugung 9 (Biotek).

Nach Einführung der Spitze des Katheters 1 in die Nähe der Referenzdruckmessung, wird das Rohr 7 mit einem definierten Frequenzgemisch-Druck angeregt. In getrennten Messungen wird der Mitteldruck, üblicherweise im Bereich von 0 mmHg bis 130 mmHg in definierten äquidistanten Schrittweiten variiert. Der Frequenzinhalt des Anregungssignals setzt sich aus einer Grundschwingung und mehreren harmonischen Oberschwingungen zusammen. Die Grundschwingung beträgt üblicherweise 0,25 Hz und es werden 160 harmonische Oberschwingungen angeregt, so dass durch die äquidistante Abstände eine obere Frequenz von 40 Hz erreicht wird. Selbstverständlich sind andere Frequenzen der Grundschwingung möglich, ebenso wie die Anzahl der harmonischen Oberschwingungen variiert werden kann. Die genannten Werte stellen jedoch eine sinnvolle Auswahl dar.

Aus jeder Messung wird das Fourier-Spektrum des Referenzsignals und des Flüssigkeitsdrucksignals mittels Fourier-Transformation berechnet. Der Korrekturdatensatzvektor ergibt sich dann aus der komplexen Division jeder Spektrallinie des Referenzdrucks durch die entsprechende Spektrallinie des Flüssigkeitsdrucks. Das Ergebnis ist ein einheitenloser, komplexer Korrekturfaktor für jede Spektrallinie dieser Messung. Alle Messungen zusammen ergeben die Korrekturdatensatzmatrix für das untersuchte System, die in der Signalanalyse- und -verarbeitungseinheit 5 gespeichert werden.

Bei der schlagweisen Analyse entspricht die Grundschwingungslänge bei der invasiven Druckmessung einem Herzschlag, wobei sich die Herzfrequenz von Schlag zu Schlag stark ändern kann. Eine laufende Analyse der Grundfrequenz ist deshalb Teil der automatischen Signalanalyse und wird durch eine Autokorrelationsfunktion bestimmt.

Bei der segmentweisen Analyse der aufgenommenen Signale wird mit unterschiedlichen Segmentlängen gearbeitet, die sich aus einem Vergleich der Rücktransformierten mit dem Originalsignal ergeben, wobei die Segmentlängen zweckmäßigerweise so gewählt werden, dass ein minimaler Fehler vorliegt, der erfahrungsgemäß bei ca. 1% liegt. Dies bedeutet, dass die Vorrichtung für die Korrektur eine optimale Segmentlänge ermittelt hat.

Die Anzahl der Fourierkoeffizienten und damit der Korrekturdatensatz sind somit abhängig von der Länge des analysierten Segmentes bzw. der Grundfrequenz.

Neben der Ermittlung der Grundfrequenz bzw. der Segmentlänge ist der Mitteldruck eine zu ermittelnde Größe. Die Systemübertragungseigenschaften des flüssigkeitsgefüllten Systems sind u.a. abhängig von der Elastizität des Katheter- und Leitungssystems 1, 2. Abhängig von den Materialeigenschaften kann eine unterschiedliche Vorspannung durch den im System herrschenden mittleren Binnendruck die Systemübertragungseigenschaften deshalb wesentlich verändern. Eine laufende Messung des Mitteldrucks ist deshalb ebenfalls Teil der automatischen Signalanalyse. Die Auswahl der Korrekturdatensätze erfolgt abhängig vom Mitteldruck.

Vor Beginn der eigentlichen Korrektur kann das Signal frequenzgefiltert werden, wobei für den numerischen Filter ein wahlweiser Einsatz vorgesehen ist, ebenso wie eine Variation der Filtergrenzfrequenz, die erfahrungsgemäß zwischen 40 und 100 Hz liegt. Eine solche Filterung kann beispielsweise bei Störungen notwendig sein, die durch das 50 Hz-Wechseistromnetz hervorgerufen werden.

Zur Charakterisierung des Signals für die Korrektur werden die Grundfrequenz bzw. die Segmentlänge und der Mitteldruck benötigt.

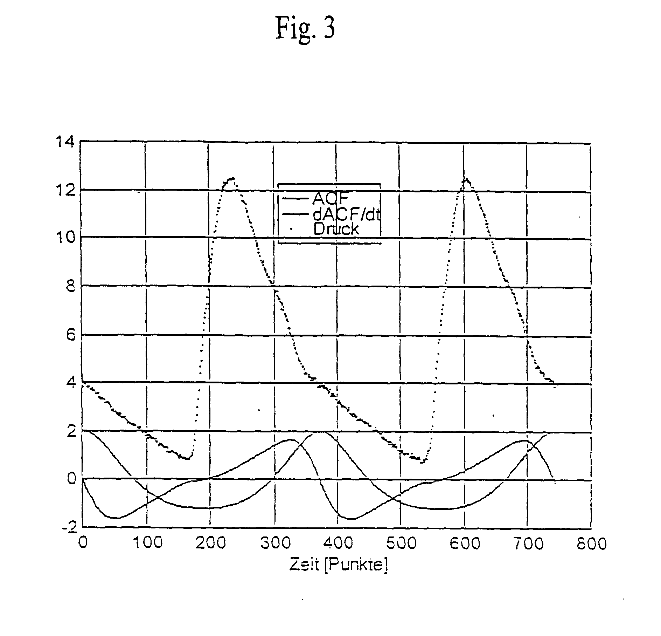

In Figur 3 ist ein Beispiel zur Ermittlung der Grundschwingungslänge dargestellt. Dazu wird zunächst die Autokorrelationsfunktion (ACF) berechnet. Die Zeit bis zum Auftreten eines einen Schwellwert überschreitenden Hauptmaximums ist die Grundschwingungslänge.

Nach der Höhe des Mitteldruckes wird der entsprechende Korrekturdatensatz ausgewählt. Der Mitteldruck ergibt sich aus der normierten Höhe der ersten Spektrallinie (Linie der Frequenz Null, Gleichanteil) der Fouriertransformierten des Signals.

Zusätzlich wird einer möglichen Abhängigkeit der Übertragungscharakteristik des Systems vom Frequenzinhalt des anregenden Signals durch eine einfache Formanalyse des Signals, basierend auf höheren harmonischen Grundschwingungen mit entsprechender Modifikation der Korrekturdatensätze, begegnet.

In einer bevorzugten Alternative werden die komplexen Fourierkoeffizienten des Drucksignals dann mit den komplexen Korrekturkoeffizienten des ausgewählten Korrekturvektors multipliziert. Ähnlich wie bei der Erstellung der Korrekturdatensätze werden bei der schlagweisen Analyse auch beim Drucksignal nur die Grundfrequenz und ihre harmonischen Oberschwingungen, soweit sie einen Schwellwert überschreiten, bis zu einer oberen Frequenz entsprechend der höchsten Frequenz der Korrekturdatensätze, im vorliegenden Fall 40 Hz, korrigiert. Alle anderen Frequenzanteile werden auf Null gesetzt.

Aus der Multiplikation entsteht das korrigierte Fourierspektrum des Drucksignals, dass dann mittels inverser diskreter Fouriertransformation in das korrigierte Drucksignal rücktransformiert wird.

In einer anderen Ausgestaltungsform der Erfindung wird bei der schlagweisen Analyse aus den Größen Grundfrequenz und Mitteldruck der entsprechende Korrekturdatensatz aus der Korrekturdatensatzmatrix ausgewählt. Liegt die Position der Messung nicht exakt auf einem Koordinatenpunkt der Matrix, so werden alle Koeffizienten mit einer gewichteten Interpolation aus den benachbarten Koeffizienten neu berechnet.

Der Kehrwert der Grundfrequenz, die Grundschwingungslänge, bestimmt die Anzahl der Punkte für die nachfolgende diskrete Fouriertransformation des Drucksignals, wobei das zu korrigierende Segment für die Fourieranalyse nach Bedarf verdoppelt oder vervielfacht wird. Die komplexen Fourierkoeffizienten des Drucksignals werden dann mit den komplexen Korrekturkoeffizienten des ausgewählten oder des interpolierten Korrekturvektors multipliziert.

Um eine Übereinstimmung der Spektrallinien des zu korrigierenden Drucksignals mit denen des Korrekturdatensatzvektors zu erreichen, wird das Drucksignalsegment (hier ein Herzschlag) so oft vervielfacht, bis sich ein Verhältnis zwischen der Abtastrate und der Kurvenstücklänge ergibt, welches der Auflösung des Korrekturdatensatzes entspricht.

Wenn beispielsweise Korrekturkoeffizienten für die Frequenzen 0,25 Hz, 0,50 Hz, 0,75 Hz, ... 40 Hz (spektrale Auflösung 0,25 Hz) vorliegen, muss bei einer Abtastrate von 1000 Hz das Kurvenstück des Drucksignals mindestens 4000 Punkte enthalten, denn dann ergibt sich ein Verhältnis von Abtastrate zu Kurvenstücklänge von 1/4 (<=> 0,25 Hz). Lässt sich dieses Verhältnis nicht exakt erreichen, wird das nächst kleinere Verhältnis (<1/4) eingestellt. Die Zuordnung zu den Spektrallinien des Korrekturdatensatzes erfolgt dann durch Aufrunden zur nächsten entsprechenden Linie.

Zur Bestimmung der Grundfrequenz wird eine Verteilungsanalyse von Maxima von Autokorrelationsfunktionen variierender Länge mit der Analyse der Minima und Maxima der Kurve kombiniert.

Zur online-Bestimmung der Grundfrequenz eines Drucksignals berechnet die Signalsanalyse- und -verarbeitungseinheit 5 der Vorrichtung die Grundfrequenz mittels Autokorrelationsfunktion (ACF). Dabei entspricht die Anzahl der Funktionswerte bis zum ersten Hauptmaximum der Länge des Schlages, also dem Kehrwert der Grundfrequenz. Da im online-Betrieb die Anzahl der Messwerte zu Beginn klein ist und mit der Zeit wächst, wird die ACF mit wachsender Länge wiederholt. Hierbei ergibt sich das Problem, dass ein stark veränderter zweiter Schlag das Ergebnis stark beeinflusst. Zur optimalen Entscheidung, wann die Schlaglänge richtig bestimmt wurde, werden alle ersten Maxima der ACF's größer werdender Länge gesammelt und mittels einer Verteilungsanalyse wird das Maximum ausgewählt, welches am häufigsten vorkommt.

Bei einer segmentweisen Analyse des gemessenen Signals kann auf eine Bestimmung der Grundfrequenz mittels der Autokorrelation verzichtet werden. Zur Segmentlängenbestimmung wird für eine Mindestlänge, z.B. 0.3 Sekunden, des digitalisierten Drucksignals das komplexe Fourier-Spektrum berechnet. Die Frequenzanteile oberhalb einer festgelegten Grenze, die durch die höchste Frequenz der Korrekturdatensätze bestimmt ist, im vorliegenden Fall 40 Hz, werden auf Null gesetzt. Anschließend wird das Spektrum in den Zeitbereich zurück transformiert und punktweise mit der Originalkurve verglichen. Der Vergleich ergibt eine Abweichung mit einem bestimmten Wert. Die Länge des untersuchten Segmentes wird nun schrittweise vergrößert und die Transformation, Frequenzfilterung, Rücktransformation und Abweichungsbestimmung werden solange wiederholt, bis ein Minimum der Abweichung gefunden wurde. Die so ermittelte Segmentlänge ist optimal für die durch die Signalanalyse- und -verarbeitungseinheit 5 der Vorrichtung durchgeführte Fouriertransformation, die sich an die Segmentlängenbestimmung anschließt.

Ein mit 1000 Punkten pro Sekunde abgetastetes Signal für den Korrekturdatensatz wird mit einer 4000 Punkte Fouriertransformation behandelt. Daraus ergibt sich:
f₁ = 0 Hz, f₂ = 0.5 Hz, u.s.w. bis fₙ = 40 Hz

Wird das zu korrigierende Kurvenstück ebenfalls mit 1000 Punkten pro Sekunde abgetastet und ist die Grundschwingungslänge 1000 Punkte lang, so ergeben sich die Frequenzen der Fouriertransformation zu:
h₁ = 0 Hz, h₂ = 1 Hz, h₃ = 2 Hz u.s.w. bis hₘ = 999 Hz

Um die 160 Punkte der Fouriertransformation des Korrekturdatensatzes auf die 1000 Punkte des Kurvensegmentes unter Beibehaltung eines stetigen Druckverlaufes anwenden zu können, werden die sich entsprechenden Frequenzlinien bis 40 Hz zur Korrektur verwendet und mit den Werten des Kurvensegmentes multipliziert. Alle, übrigen Frequenzlinien werden auf Null gesetzt. Aus der Multiplikation entsteht das korrigierte Fourierspektrum des Drucksignals, das dann mittels inverser diskreter Fouriertransformation in das korrigierte Drucksignal rücktransformiert wird.

Für Nachbearbeitungsvorgänge kann, wie bei dem Signaleingang, das Ausgangssignal ebenfalls frequenzgefiltert werden. Der numerische Filter kann vom Anwender wahlweise an- und ausgeschaltet sowie die Filtergrenzfrequenz variiert werden. Eine Signalverbesserung wird auch durch eine an die Frequenzfilterung angeschlossene Mittelwertfilterung erreicht, weshalb ein frei konfigurierbarer Mittelwertfilter (Moving Average Filter) mit einer Länge von 2 bis 20 Punkten vorgesehen ist. Auch dieser Filter kann ein- oder ausgeschaltet werden. Zur Verbesserung des Korrekturergebnis kann eine Zusatzkorrektur eingeschaltet werden, die die um n Punkte verschobene erste Ableitung nach der Zeit zum korrigierten Signal punktweise addiert oder subtrahiert.

## Patentansprüche

1. Vorrichtung zur Korrektur von Meßwertverfälschungen bei invasiven Druckmessungen mit einem flüssigkeitsgefüllten System,
mit einem Druckwandler (3) zur Umwandlung der aufgenommenen Drücke in elektrische Signale,
einem mit dem Druckwandler (3) verbundenen Analog-Digitalwandler (4) zur Digitalisierung der elektrischen Signale und
einer an den Analog-Digitalwandler (4) angeschlossenen Signalanalyse- und -verarbeitungseinheit (5) sowie
einer an die Signalanalyse- und -verarbeitungseinheit (5) angeschlossene Ausgabe- bzw. Auswerteeinheit (6),
**dadurch gekennzeichnet, daß**
die Signalanalyse- und -verarbeitungseinheit (5)
- Mittel zur Durchführung einer Fourieranalyse aufweist, die das Signal in Segmente zerlegt und diese einer Fourieranalyse unterzieht,
- das segmentierte Signal mit vorgebbaren Korrekturdaten in Form von Fourierkoeffizienten verknüpft, wobei die Länge der Segmente so variiert wird, daß ein minimaler Fehler bei der Fourieranalyse auftritt und
- das korrigierte Signal an die Ausgabe- und/ oder Auswerteeinheit (6) abgibt.

2. Vorrichtung nach Anspruch 1, **dadurch gekennzeichnet, daß** die Signalanalyse- und -verarbeitungseinheit (5) die Segmentlänge des zu korrigierenden Signals durch eine Variation der Länge eines Basissignals und einen Vergleich der Rücktransformierten des Basissignals mit dem Originalsignal bestimmt, wobei die Abweichung der Rücktransformierten von dem Originalsignal einen vorgegebenen Wert einnimmt.

3. Vorrichtung nach Anspruch 2, **dadurch gekennzeichnet, daß** die Signalanalyse- und -verarbeitungseinheit (5) die Segmentlänge durch das Minimum der Abweichung der Rücktransformierten von dem Originalsignal bestimmt.

4. Vorrichtung nach Anspruch 2 oder 3, **dadurch gekennzeichnet, daß** die Signalanalyse- und -verarbeitungseinheit (5) die Segmentlänge von einer Basissignallänge ausgehend schrittweise verändert.

5. Vorrichtung nach Anspruch 4, **dadurch gekennzeichnet, daß** die Signalanalyse- und -verarbeitungseinheit (5) die Segmentlänge von einer Mindestlänge ausgehend schrittweise vergrößert.

6. Vorrichtung nach einem der Ansprüche 2 bis 5, **dadurch gekennzeichnet, daß** die Signalanalyse- und -verarbeitungseinheit (5) die Schrittweite der Segmentlängenänderung proportional zu der Abweichung der Rücktransformierten von dem Originalsignal variiert.

7. Vorrichtung zur Korrektur von Meßwertverfälschungen bei : invasiven Druckmessungen mit einem flüssigkeitsgefüllten System,
mit einem Druckwandler (3) zur Umwandlung der aufgenommenen Drücke in elektrische Signale,
einem mit dem Druckwandler (3) verbundenen Analog-Digitalwandler (4) zur Digitalisierung der elektrischen Signale und
einer an den Analog-Digitalwandler (4) angeschlossenen Signalanalyse- und -verarbeitungseinheit (5), der das digitalisierte Signal zuführbar ist, sowie
einer an die Signalanalyse- und- verarbeitungseinheit (5) angeschlossene Ausgabe- bzw. Auswerteeinheit (6)
**dadurch gekennzeichnet, daß**
die Signalanalyse- und -verarbeitungseinheit (5)
- Mittel zur Durchführung einer Fourieranalyse aufweist, die das Signal in Segmente der Länge eines Herzschlags zerlegt und das Signal so schlagweise einer Fourieranalyse unterzieht,
- das Signal auf der Grundlage der schlagweisen Analyse mit vorgebbaren Korrekturdaten in Form von Fourierkoeffizienten verknüpft, wobei die der Herzfrequenz entsprechende Grundfrequenz über eine Autokorrelationsfunktion und deren erste Ableitung nach der Zeit ermittelt wird, und
- das korrigierte Signal an die Ausgabe- und/ oder Auswerteeinheit (6) abgibt.

8. Vorrichtung nach mindestens einem der voranstehenden Ansprüche, **dadurch gekennzeichnet, daß** die Signalanalyse- und -verarbeitungseinheit (5) die Korrekturdaten der Meßwerte aus einer Korrekturdatensatzmatrix, vorzugsweise als Korrekturdatensatzvektor abruft.

9. Vorrichtung nach mindestens einem der voranstehenden Ansprüche, **dadurch gekennzeichnet, daß** die Signalanalyse- und -verarbeitungseinheit (5) eine Phasen- und/oder Amplitudenkorrektur des Signals vorzugsweise nur an den Stellen, wo das Signal eine Amplitude aufweist, durchführt.

10. Vorrichtung nach mindestens einem der voranstehenden Ansprüche, **dadurch gekennzeichnet, daß** die Signalanalyse- und -verarbeitungseinheit (5) zwischen den Korrekturdatensätzen eine Interpolation durchführt.

11. Vorrichtung nach mindestens einem der voranstehenden Ansprüche, **gekennzeichnet durch** eine druckbeaufschlagbare Vorrichtung (7) zur Ermittlung der Korrekturdatensatzmatrix, in die das flüssigkeitsgefüllte System einführbar ist und die mit unterschiedlichen Mitteldrücken und Frequenzen beaufschlagbar ist, und mit der eine Referenzdruckmessung mit einem unterschiedlichen Meßsystem (10) erfolgt sowie eine harmonische Analyse durchgeführt wird.

12. Vorrichtung nach mindestens einem der voranstehenden Ansprüche, **dadurch gekennzeichnet, daß** die Signalanalyse- und -verarbeitungseinheit (5) zur Ermittlung der Korrekturdatensätze für die Systemanregung ein auf einer Grundschwingung zwischen 0,2 Hz und 3 Hz und harmonischen Oberschwingungen basierendes Frequenzgitter verwendet.

13. Vorrichtung nach Anspruch 12, **dadurch gekennzeichnet, daß** eine entsprechende Anzahl Oberschwingungen angeregt wird, bis durch äquidistante Abstände eine festgelegte Obergrenze von vorzugsweise 40 Hz erreicht ist.

14. Vorrichtung nach mindestens einem der voranstehenden Ansprüche, **dadurch gekennzeichnet, daß** das Drucksignalsegment so oft vervielfacht wird, bis sich ein Verhältnis zwischen der Abtastrate und der Kurvenstücklänge ergibt, das der Auflösung des Korrekturdatensatzes entspricht, um eine Übereinstimmung der Spektrallinien des zu korrigierenden Signals mit denen des Korrekturdatensatzvektors zu erreichen.

15. Vorrichtung nach Anspruch 14, **dadurch gekennzeichnet, daß** bei Nichtentsprechen der Auflösung das nächstkleinere Verhältnis zwischen der Abtastrate und der Kurvenstücklänge eingestellt wird und die Zuordnung zu den Spektrallinien des Korrekturdatensatzes durch Aufrunden zur nächsten entsprechenden Linie erfolgt.

16. Vorrichtung nach Anspruch 11, **dadurch gekennzeichnet, daß** die Übertragungscharakteristik des flüssigkeitsgefüllten Systems mittels eines weißen Frequenzrauschens bestimmt und die Korrektur mittels Dekonvolution des Ausgangssignals mit der Übertragungsfunktion durchgeführt wird.

17. Vorrichtung nach mindestens einem der voranstehenden Ansprüche, **dadurch gekennzeichnet, daß** zwischen dem Druckwandler (3) und dem Analog-Digitalwandler (4) ein Verstärker angeordnet ist.

18. Vorrichtung nach mindestens einem der vorangegangenen Ansprüche, **dadurch gekennzeichnet, daß** die Signalanalyse- und -verarbeitungseinheit (5) den Druckwandler (3) ansteuert und mit Spannung versorgt.

19. Vorrichtung nach mindestens einem der Ansprüche 7 bis 18, **dadurch gekennzeichnet, daß** die Grundfrequenz über eine Autokorrelationsfunktion und deren erste Ableitung nach der Zeit ermittelt wird.

20. Vorrichtung nach mindestens einem der Ansprüche 7 bis 18, **dadurch gekennzeichnet, daß** die Grundfrequenz mittels einer Kombination einer Verteilungsanalyse von Maxima von Autokorrelationsfunktionen variierender Länge mit der Analyse der Minima und Maxima der Kurve bestimmt wird.

21. Vorrichtung nach Anspruch 20, **dadurch gekennzeichnet, daß** zur online-Bestimmung der Grundfrequenz die Autokorrelationsfunktion mit wachsender Länge wiederholt wird und alle ersten Maxima der Autokorrelationsfunktionen größer werdender Länge gesammelt werden und mittels einer Verteilungsanalyse das am häufigsten vorkommende Maximum ermittelt wird.

22. Vorrichtung nach mindestens einem der Ansprüche 7 bis 21, **dadurch gekennzeichnet, daß** zur Ermittlung der Signallaufzeit eine Kreuzkorrelation von Drucksignal und Patienten-EKG durchgeführt wird.

23. Vorrichtung nach mindestens einem der voranstehenden Ansprüche, **dadurch gekennzeichnet, daß** eine Systemidentifikation über eine Testsignalantwort oder automatisch durchgeführt wird.

24. Vorrichtung nach Anspruch 23, **gekennzeichnet durch** eine manuelle Interaktion bei der Systemidentifikation.

25. Vorrichtung nach mindestens einem der voranstehenden Ansprüche, **gekennzeichnet durch** eine laufende Messung des Mitteldruckes.

26. Vorrichtung nach Anspruch 23, **dadurch gekennzeichnet, daß** der Druckwandler (3) oder ein Kalibrator ein Testsignal generiert.

27. Vorrichtung nach mindestens einem der voranstehenden Ansprüche, **gekennzeichnet durch** eine Identifikation von Artefakten auf der Basis von Systemidentifikation, der harmonischen Basisfrequenz und des Signalmitteldruckes.

28. Vorrichtung nach Anspruch 27, **gekennzeichnet durch** eine Artefaktidentifikation und -elimination mittels einer Autokorrelationsfunktion und **durch** eine Interpolation der Meßwerte an der Stelle eines Spikes zur Glättung einer Störung.

29. Vorrichtung nach mindestens einem der voranstehenden Ansprüche, **gekennzeichnet durch** eine Analyse der Grundfrequenz, des Mitteldruckes und der Form des Drucksignals mittels harmonischer Analyse.

30. Vorrichtung nach Anspruch 29, **gekennzeichnet durch** eine Korrektur unter Berücksichtigung von höheren harmonischen Grundschwingungen.

31. Vorrichtung nach mindestens einem der voranstehenden Ansprüche, **dadurch gekennzeichnet, daß** die Ausgabe- oder Auswerteeinheit (6) das untransformierte Signal ausgibt.

32. Vorrichtung nach mindestens einem der voranstehenden Ansprüche, **gekennzeichnet durch** eine Kalibrierung des Meßsystems in Form eines Nullpunktabgleiches, einer Referenzdruckmessung und/oder eines Testsignals.

33. Vorrichtung nach mindestens einem der voranstehenden Ansprüche, **dadurch gekennzeichnet, daß** die Signalanalyse- und -verarbeitungseinheit (5) das Signal mittels Frequenz- und/oder Mittelwertfilter nachfiltert und/oder basierend auf der ersten Ableitung des Drucksignals nach der Zeit nachkorrigiert.

34. Vorrichtung nach mindestens einem der voranstehenden Ansprüche, **gekennzeichnet durch** eine automatische Anpassung an Änderungen des Resonanzverhaltens infolge von Druckänderungen.

35. Vorrichtung nach mindestens einem der voranstehenden Ansprüche, **dadurch gekennzeichnet, daß** zwischen der Signalanalyse- und -verarbeitungseinheit (5) und der Ausgabe bzw. Auswerteeinheit (6) eine als Digital-Analogwandler, Verstärker und/oder Adapter ausgebildete Schnittstelle vorgesehen ist.

36. Vorrichtung nach mindestens einem der voranstehenden Ansprüche, **dadurch gekennzeichnet, daß** eine Korrekturdatensatzmatrix in einem Speicher der Signalanalyse-und -verarbeitungseinheit (5) abgelegt ist.

37. Vorrichtung nach einem der vorangehenden Ansprüche, **gekennzeichnet durch** einen Signalausgang für das unkorrigierte Signal.

38. Vorrichtung nach einem der vorangehenden Ansprüche, **dadurch gekennzeichnet, daß** eine Vorrichtung zur Messung von Blutdruckschwankungen vorgesehen ist.

## Claims

1. Device for the correction of measured value falsifications in invasive pressure measurements with a fluid-filled system, with a pressure transducer (3) for converting the recorded pressures into the electrical signals,
an analogue/digital converter (4), connected to the pressure transducer (3), for digitising the electrical signals and
a signal analysing and processing unit (5), connected to the analog/digital converter (4), and
an output and/or evaluating unit (6), connected to the signal analysing and processing unit (5),
**characterized in that**
the signal analysing and processing unit (5)
- has means for carrying out a Fourier analysis, which breaks down the signal into segments and subjects them to a Fourier analysis,
- links the segmented signals with predeterminable correction data in the form of Fourier coefficients, the length of the segments being varied such that a minimal error occurs in the Fourier analysis, and
- emits the corrected signal to the output and/or evaluation unit (6).

2. Device according to Claim 1, **characterized in that** the signal analysing and processing unit (5) determines the segment length of the signal to be corrected by a variation of the length of a base signal and a comparison of the inverse transform of the base signal with the original signal, the deviation of the inverse transform from the original signal assuming a prescribed value.

3. Device according to Claim 2, **characterized in that** the signal analysing and processing unit (5) determines the segment length by the minimum of the deviation of the inverse transform from the original signal.

4. Device according to Claim 2 or 3, **characterized in that** the signal analysing and processing unit (5) changes the segment length in steps, starting from a base signal length.

5. Device according to Claim 4, **characterized in that** the signal analysing and processing unit (5) increases the segment length in steps, starting from a minimum length.

6. Device according to one of Claims 2 to 5, **characterized in that** the signal analysing and processing unit (5) varies the step size of the segment length change in proportion to the deviation of the inverse transform from the original signal.

7. Device for the correction of measured value falsifications in invasive pressure measurements with a fluid-filled system, with a pressure transducer (3) for converting the recorded pressures into the electrical signals,
an analogue/digital converter (4), connected to the pressure transducer (3), for digitising the electrical signals and
a signal analysing and processing unit (5), connected to the analog-digital converter (4), to which unit the digitised signal can be fed, and
an output and/or evaluating unit (6), connected to the signal analysing and processing unit (5),
**characterized in that**
the signal analysing and processing unit (5)
- has means for carrying out a Fourier analysis, which breaks down the signal into segments the length of a heartbeat and subjects the signal in this way beat by beat to a Fourier analysis,
- links the signal on the basis of the beat-related analysis with predeterminable correction data in the form of Fourier coefficients, the fundamental frequency corresponding to the heart rate being determined by means of an autocorrelation function and its first derivative with respect to time, and
- emits the corrected signal to the output and/or evaluation unit (6).

8. Device according to at least one of the preceding claims, **characterized in that** the signal analysing and processing unit (5) calls up the correction data of the measured values from a correction data record matrix, preferably as a correction data record vector.

9. Device according to at least one of the preceding claims, **characterized in that** the signal analysing and processing unit (5) carries out a phase and/or amplitude correction of the signal, preferably only at the points where the signal has an amplitude.

10. Device according to at least one of the preceding claims, **characterized in that** the signal analysing and processing unit (5) carries out an interpolation between the correction data records.

11. Device according to at least one of the preceding claims, **characterized by** a device (7) which can be subjected to pressure for determining the correction data record matrix into which the fluid-filled system can be introduced and which can be subjected to different medium pressures and frequencies, and with which a reference pressure measurement takes place with a different measuring system (10) and a harmonic analysis is carried out.

12. Device according to at least one of the preceding claims, **characterized in that** the signal analysing and processing unit (5) uses a frequency grid based on a fundamental oscillation between 0.2 Hz and 3 Hz and harmonic oscillations for determining the correction data records for the system excitation.

13. Device according to Claim 12, **characterized in that** a corresponding number of harmonic oscillations are excited until a fixed upper limit of preferably 40 Hz is reached by equidistant intervals.

14. Device according to at least one of the preceding claims, **characterized in that** the pressure signal segment is repeated until a ratio which corresponds to the resolution of the correction data record is obtained between the sampling rate and the length of the curve segment, in order to achieve a coincidence of the spectral lines of the signal to be corrected with those of the correction data record vector.

15. Device according to Claim 14, **characterized in that**, if it does not correspond to the resolution, the next-smaller ratio between the sampling rate and the length of the curve segment is set and the assignment to the spectral lines of the correction data record takes place by rounding up to the next corresponding line.

16. Device according to Claim 11, **characterized in that** the transmission characteristic of the fluid-filled system is determined by means of a white frequency noise and the correction is carried out by means of deconvolution of the output signal with the transmission function.

17. Device according to at least one of the preceding claims, **characterized in that** an amplifier is arranged between the pressure transducer (3) and the analogue/digital converter (4).

18. Device according to at least one of the preceding claims, **characterized in that** the signal analysing and processing unit (5) activates the pressure transducer (3) and supplies it with voltage.

19. Device according to at least one of Claims 7 to 18, **characterized in that** the fundamental frequency is determined by means of an autocorrelation function and the first derivative with respect to time of the latter.

20. Device according to at least one of Claims 7 to 18, **characterized in that** the fundamental frequency is determined by means of a combination of a distribution analysis of maxima of autocorrelation functions of varying length with the analysis of the minima and maxima of the curve.

21. Device according to Claim 20, **characterized in that**, for online determination of the fundamental frequency, the autocorrelation function is repeated with an increasing length and all the first maxima of the autocorrelation functions of the increasing length are collected and the most frequently occurring maximum is determined by means of a distribution analysis.

22. Device according to at least one of Claims 7 to 21, **characterized in that**, for determining the signal delay time, a cross-correlation of the pressure signal and patient's ECG is carried out.

23. Device according to at least one of the preceding claims, **characterized in that** a system identification is carried out by means of a test signal response or automatically.

24. Device according to Claim 23, **characterized by** a manual interaction in the system identification.

25. Device according to at least one of the preceding claims, **characterized by** a continuous measurement of the medium pressure.

26. Device according to Claim 23, **characterized in that** the pressure transducer (3) or a calibrator generates a test.

27. Device according to one of the preceding claims, **characterized by** an identification of artefacts on the basis of system identification, the harmonic base frequency and the signal medium pressure.

28. Device according to Claim 27, **characterized by** an artefact identification and elimination by means of an autocorrelation function and by an interpolation of the measured values at the point of a spike for smoothing an interference.

29. Device according to at least one of the preceding claims, **characterized by** an analysis of the fundamental frequency, the medium pressure and the shape of the pressure signal by means of harmonic analysis.

30. Device according to Claim 29, **characterized by** a correction, taking higher harmonic fundamental oscillations into account.

31. Device according to at least one of the preceding claims, **characterized in that** the output or evaluating unit (6) outputs the untransformed signal.

32. Device according to at least one of the preceding claims, **characterized by** a calibration of the measuring system in the form of a zero point calibration, a reference pressure measurement and/or a test signal.

33. Device according to at least one of the preceding claims, **characterized in that** the signal analysing and processing unit (5) post-filters the signal by means of frequency and/or mean-value filter and/or post-corrects the signal on the basis of the first derivative of the pressure signal with respect to time.

34. Device according to at least one of the preceding claims, **characterized by** an automatic adaptation to changes of the resonant response as a result of pressure changes.

35. Device according to at least one of the preceding claims, **characterized in that** an interface designed as a digital/analogue converter, amplifier and/or adaptor is provided between the signal analysing and processing unit (5) and the output and/or evaluating unit (6).

36. Device according to at least one of the preceding claims, **characterized in that** a correction data record matrix is stored in a memory of the signal analysing and processing unit (5).

37. Device according to at least one of the preceding claims, **characterized by** a signal output for the uncorrected signal.

38. Device according to at least one of the preceding claims, **characterized in that** a device for measuring blood pressure fluctuations is provided.

## Revendications

1. Dispositif de correction d'erreurs sur des valeurs mesurées dans le cas de mesures de pression invasives avec un système rempli d'un fluide,
comprenant un convertisseur de pression (3) destiné à convertir les pressions reçues en signaux électriques,
un convertisseur analogique-numérique (4) relié au convertisseur de pression (3), destiné à numériser les signaux électriques et
une unité d'analyse et de traitement des signaux (5) raccordée au convertisseur analogique-numérique (4) ainsi
qu'une unité de sortie et/ou d'exploitation (6) raccordée à l'unité d'analyse et de traitement des signaux (5),
**caractérisé en ce que**
l'unité d'analyse et de traitement des signaux (5)
- comprend des moyens destinés à réaliser une analyse de Fourier, qui décompose le signal en segments et soumet ces derniers à une analyse de Fourier,
- combine le signal segmenté aux données correctrices pouvant être prédéfinies sous forme de coefficients de la série de Fourier, la longueur des segments variant de sorte qu'une erreur minimale se produit à l'occasion de l'analyse de Fourier, et
- délivre le signal corrigé à l'unité de sortie et/ou d'exploitation (6).

2. Dispositif selon la revendication 1, **caractérisé en ce que** l'unité d'analyse et de traitement des signaux (5) définit la longueur de segment du signal destiné à être corrigé par l'intermédiaire d'une variation de la longueur d'un signal de base et une comparaison des transformées inverses du signal de base au signal original, l'écart entre la transformée inverse et le signal original prenant une valeur prédéfinie.

3. Dispositif selon la revendication 2, **caractérisé en ce que** l'unité d'analyse et de traitement des signaux (5) définit la longueur de segment par l'intermédiaire du minimum de l'écart entre la transformée inverse et le signal original.

4. Dispositif selon la revendication 2 ou 3, **caractérisé en ce que** l'unité d'analyse et de traitement des signaux (5) modifie progressivement la longueur de segment à partir d'une longueur de signal de base.

5. Dispositif selon la revendication 4, **caractérisé en ce que** l'unité d'analyse et de traitement des signaux (5) augmente progressivement la longueur de segment à partir d'une longueur minimale.

6. Dispositif selon l'une quelconque des revendications 2 à 5, **caractérisé en ce que** l'unité d'analyse et de traitement des signaux (5) fait varier l'incrément de la modification de la longueur du segment de façon proportionnelle à l'écart entre la transformée inverse et le signal original.

7. Dispositif de correction d'erreurs sur des valeurs mesurées à l'occasion de mesures de pression invasives avec un système rempli d'un fluide,
comprenant un convertisseur de pression (3) destiné à convertir les pressions reçues en signaux électriques,
un convertisseur analogique-numérique (4) relié au convertisseur de pression (3), destiné à numériser les signaux électriques et
une unité d'analyse et de traitement des signaux (5) raccordée au convertisseur analogique-numérique (4), à laquelle le signal numérisé peut être amené, ainsi
qu'une unité de sortie et/ou d'exploitation (6) raccordée à l'unité d'analyse et de traitement des signaux (5),
**caractérisé en ce que**
l'unité d'analyse et de traitement des signaux (5)
- comprend des moyens destinés à réaliser une analyse de Fourier, qui décompose le signal en segments de la longueur d'un battement de coeur et soumet ainsi le signal de façon cadencée à une analyse de Fourier,
- combine le signal sur la base de l'analyse cadencée aux données correctrices pouvant être prédéfinies sous forme de coefficients de la série de Fourier, la fréquence fondamentale correspondant à la fréquence cardiaque étant déterminée par l'intermédiaire d'une fonction d'autocorrélation et par sa première dérivée par rapport au temps, et
- délivre le signal corrigé à l'unité de sortie et d'exploitation (6).

8. Dispositif selon l'une quelconque des revendications précédentes, **caractérisé en ce que** l'unité d'analyse et de traitement des signaux (5) appelle les données correctrices des valeurs mesurées à partir d'une matrice d'enregistrement de correction, de préférence comme vecteur d'enregistrement de correction.

9. Dispositif selon l'une quelconque des revendications précédentes, **caractérisé en ce que** l'unité d'analyse et de traitement des signaux (5) réalise une correction de phase et/ou d'amplitude du signal, de préférence uniquement aux points où le signal comprend une amplitude.

10. Dispositif selon l'une quelconque des revendications précédentes, **caractérisé en ce que** l'unité d'analyse et de traitement des signaux (5) réalise une interpolation entre les enregistrements de correction.

11. Dispositif selon l'une quelconque des revendications précédentes, **caractérisé par** un dispositif pouvant être pressurisé (7) destiné à déterminer la matrice d'enregistrement de correction, dans lequel peut être introduit le système rempli de fluide et qui peut être sollicité par les différentes pressions moyennes et fréquences, et avec lequel une mesure de pression de référence est obtenue avec un système de mesure (10) différent et une analyse harmonique est réalisée.

12. Dispositif selon l'une quelconque des revendications précédentes, **caractérisé en ce que** l'unité d'analyse et de traitement des signaux (5) destinée à déterminer les enregistrements de correction pour l'excitation du système utilise une gigue de fréquence se basant sur une composante fondamentale entre 0,2 Hz et 3 Hz et sur des composantes harmoniques.

13. Dispositif selon la revendication 12, **caractérisé en ce qu'**un nombre correspondant de composantes harmoniques est excité, jusqu'à ce qu'un plafond fixé, de préférence de 40 Hz, soit atteint par intervalles équidistants.

14. Dispositif selon l'une quelconque des revendications précédentes, **caractérisé en ce que** le segment de signal de pression est multiplié jusqu'à ce qu'il se produise un rapport entre le taux d'échantillonnage et la longueur de partie de courbe, qui correspond à la résolution de l'enregistrement de correction, afin d'obtenir une concordance entre les raies spectrales du signal destiné à être corrigé et celles du vecteur d'enregistrement de correction.

15. Dispositif selon la revendication 14, **caractérisé en ce que**, en cas d'absence de correspondance de la résolution, le rapport immédiatement inférieur entre le taux d'échantillonnage et la longueur de partie de courbe est ajusté et l'attribution aux raies spectrales de l'enregistrement de correction est obtenu par arrondissement à la raie suivante correspondante.

16. Dispositif selon la revendication 11, **caractérisé en ce que** la caractéristique de transmission du système rempli d'un fluide est définie au moyen d'un bruit de fréquence blanc et la correction est réalisée au moyen de la déconvolution du signal de départ avec la fonction de transmission.

17. Dispositif selon l'une quelconque des revendications précédentes, **caractérisé en ce qu'**un amplificateur est disposé entre le convertisseur de pression (3) et le convertisseur analogique-numérique (4).

18. Dispositif selon l'une quelconque des revendications précédentes, **caractérisé en ce que** l'unité d'analyse et de traitement des signaux (5) commande le convertisseur de pression (3) et l'alimente en tension.

19. Dispositif selon au moins l'une des revendications 7 à 18, **caractérisé en ce que** la fréquence fondamentale est déterminée par une fonction d'autocorrélation et sa première dérivée par rapport au temps.

20. Dispositif selon au moins l'une des revendications 7 à 18, **caractérisé en ce que** la fréquence fondamentale est définie au moyen d'une combinaison d'une analyse de répartition des maxima des fonctions d'autocorrélation de longueur variable et de l'analyse des minima et des maxima de la courbe.

21. Dispositif selon la revendication 20, **caractérisé en ce que**, pour définir en ligne la fréquence fondamentale, la fonction d'autocorrélation est répétée avec une longueur croissante et tous les premiers maxima des fonctions d'autocorrélation présentant une longueur croissante sont collectés et le maximum se produisant le plus souvent est déterminé au moyen d'une analyse de répartition.

22. Dispositif selon au moins l'une des revendications 7 à 21, **caractérisé en ce que**, pour déterminer le temps de propagation du signal, on réalise une corrélation croisée du signal de pression et de l'ECG du patient.

23. Dispositif selon l'une quelconque des revendications précédentes, **caractérisé en ce qu'**on réalise une identification du système par l'intermédiaire d'une réponse de signal d'essai ou de façon automatique.

24. Dispositif selon la revendication 23, **caractérisé par** une interaction manuelle à l'occasion de l'identification du système.

25. Dispositif selon l'une quelconque des revendications précédentes, **caractérisé par** une mesure continue de la pression moyenne.

26. Dispositif selon la revendication 23, **caractérisé en ce que** le convertisseur de pression (3) ou un étalonneur génère un signal d'essai.

27. Dispositif selon l'une quelconque des revendications précédentes, **caractérisé par** une identification des artéfacts sur la base de l'identification du système, de la fréquence de base harmonique et de la pression moyenne du signal.

28. Dispositif selon la revendication 27, **caractérisé par** une identification et une élimination d'artéfact au moyen d'une fonction d'autocorrélation et par une interpolation des valeurs mesurées au point d'un pic pour lisser une perturbation.

29. Dispositif selon l'une quelconque des revendications précédentes, **caractérisé par** une analyse de la fréquence fondamentale, de la pression moyenne et de la forme du signal de pression au moyen d'une analyse harmonique.

30. Dispositif selon la revendication 29, **caractérisé par** une correction en prenant en compte les composantes harmoniques les plus élevées.

31. Dispositif selon l'une quelconque des revendications précédentes, **caractérisé en ce que** l'unité de sortie ou d'exploitation (6) affiche le signal non transformé.

32. Dispositif selon l'une quelconque des revendications précédentes, **caractérisé par** un étalonnage du système de mesure sous forme d'un réglage du point neutre, d'une mesure de pression de référence et/ou d'un signal d'essai.

33. Dispositif selon l'une quelconque des revendications précédentes, **caractérisé en ce que** l'unité d'analyse et de traitement des signaux (5) filtre le signal au moyen de filtres de valeur de fréquence et/ou moyenne et/ou le corrige en se basant sur la première dérivée du signal de pression par rapport au temps.

34. Dispositif selon l'une quelconque des revendications précédentes, **caractérisé par** une adaptation automatique aux modifications du comportement de résonance par suite des modifications de pression.

35. Dispositif selon l'une quelconque des revendications précédentes, **caractérisé en ce qu'**il est prévu entre l'unité d'analyse et de traitement des signaux (5) et l'unité de sortie et/ou d'exploitation (6) une interface conçue comme un convertisseur analogique-numérique, un amplificateur et/ou un adaptateur.

36. Dispositif selon l'une quelconque des revendications précédentes, **caractérisé en ce qu'**une matrice d'enregistrement de correction est stockée dans une mémoire de l'unité d'analyse et de traitement des signaux (5).

37. Dispositif selon l'une quelconque des revendications précédentes, **caractérisé par** une sortie de signal pour le signal non corrigé.

38. Dispositif selon l'une quelconque des revendications précédentes, **caractérisé en ce qu'**il est prévu un dispositif pour mesurer les oscillations de la pression sanguine.
